# EUROPEAN PATENT APPLICATION

(11) **EP 4 806 421 A2**
(43) Date of publication of application: **16.09.2026**
(21) Application number: 24837551.1
(22) Date of filing: 04.11.2024
(51) Int. Cl.: A61K 9/00, A61K 47/38, A61K 47/26, A61K 9/19, A61K 47/32, A61K 47/10, A61P 35/00, A61K 9/14, A61K 36/00, A61K 31/00, A61K 9/06

(54) **PHARMACEUTICAL COMPOSITIONS OF NANOCRYSTALLIZED XANTHATIN AND USE THEREOF AS AN ANTI-TUMOR AGENT**

(30) Priority: 06.11.2023 CU 20230047
(71) Applicant: Centro de Investigación y Desarrollo de Medicamentos CIDEM, 10400 la Habana (CU)
(72) Inventor: PILOTO FERRER, Janet, La Habana 10800 (CU); SALOMÓN IZQUIERDO, Suslebys, La Habana 10700 (CU); MANSO PEÑA, Amanda, La Habana 10700 (CU); BERENGUER ROQUE, Aleksandra, La Habana (CU); OSORIA ALFONSO, Luis Alberto, Santa Cruz del Norte Mayabeque (CU); BARRIOS SARMIENTO, Maydel, Marianao La Habana (CU); GONZÁLEZ SUÁREZ, Narjara, Montréal, Québec H1L3G3 (CA); Borhane, ANNABI, Montréal, Québec J4Y 3B6 (CA); BÁRZAGA FERNÁNDEZ, Pedro Gilberto, La Habana 10900 (CU); PADRÓN YAQUIS, Alejandro Saul, Playa La Habana (CU)
(74) Representative: V.O.
(86) International application number: PCT/CU2024/050007
(87) International publication number: WO 2025/098544

(57) **Abstract**

The present invention describes the procedure for obtaining nanocrystals of isolated xanthatin from a natural source. It further describes pharmaceutical compositions comprising such nanocrystals, as well as the uses and methods of treatment in which they may be used therein.

## Description

### Field of technology

### Background of the invention

Xanthatin is a natural sesquiterpene lactone with molecular formula C15H18O3, isolated mainly from the aerial parts of species belonging to the genus Xanthium (fam. Asteraceae). Members of this genus are perennial plants distributed in America, Pakistan, India, China and Eurasia.

From the structural point of view, said compound exhibits the presence of an α-methylene-γ-butyrolactone ring fused in trans arrangement with a carbocycle of 7 carbon atoms and an unsaturated ketone side chain. Several studies with xanthanolides have shown that this structural arrangement is an essential feature in the different types of biological activities exhibited by xanthatin, among them a remarkable antiproliferative activity against different tumor cell lines.

Several reports demonstrated the effectiveness of xanthatin to significantly reduce cell proliferation and induce apoptosis in a wide variety of tumor cell lines including: ovarian carcinoma (SK-OV-3 and ES-2), melanoma (SK-MEL-2), central nervous system (XF498), colon carcinoma (HCT-15 and CT26WT), human gastric carcinoma (MKN-45), non-small cell lung cancer (A549 and H1299), breast cancer (MDA-MB-231), and cervical cancer (HeLa) cells.

Several mechanisms have been proposed for the antitumor action of xanthatins. Takeda and colleagues demonstrated that xanthatins act as a catalytic inhibitor of topoisomerase IIα, promoting DNA damage in breast cancer cells.

Furthermore, xanthatins act on the STAT3 signaling pathway and the Wnt/β-catenin pathway, which are closely related to antiproliferative effects. It has also been reported that xanthatins can negatively regulate the expression of Chk1, Chk2 and alter the phosphorylation status of CDC2, which are all key regulators of the G2/M transition. Xanthatins are also reported to have anti-mitotic potential by disrupting the metaphase to anaphase transition by acting on the formation of mitotic spindle microtubules leading to apoptotic cell death. Xanthatin has also been reported to effectively inhibit the proliferation and tumorigenicity of malignant glioma cells mainly by suppressing autophagy through activation of the PI3K/Akt/mTOR pathway. Studies of the underlying mechanisms have shown that xanthatin has anti-proliferative, anti-angiogenic and pro-apoptotic effects in several types of cancers, both *in vitro* and *in vivo.* All these studies suggest that xanthatins, isolated from *X*. *strumarium,* have a potential in cancer prevention and therapy.

Xanthatin is a lipophilic molecule that is poorly soluble in water. It can be rapidly metabolized and eliminated from the body according to pharmacokinetic studies in rats after intravenous administration.

Patent JPH09188671A (1996) refers to pharmaceutical compositions prepared by conventional methods containing xanthatin as an antitumor agent for oral use in tablet and syrup form, and for injection as an intravenous infusion. Conventional methods of preparation of injectables with poorly water-soluble active ingredients involve high amounts of cosolvents and/or surfactants which may cause anaphylactic reactions in patients.

Patent CA2688486A1 (2008) refers to the formulation of sesquiterpenes as water-insoluble antitumoral together with one or more antioxidants and one or more solubilizers selected from the group consisting of PEG400, an animal or vegetable oil (e.g. olive oil), a castor oil derivative and ethylene oxide (Cremophor RH 40) and polysorbate 80. It is known that high amounts of both organic solvents and surfactants are required to achieve solubilization of water-insoluble active ingredients such as xanthatin, which may cause adverse reactions.

Patent CN110123754A (2019) refers to obtaining a xanthatin polymeric nanomicelle functionalized with cyclic peptide (NGR) for active targeting of dentritic cells in the treatment of allergic rhinitis as an anti-inflammatory. The aqueous pharmaceutical formulation without any solubilizer or organic solvent is presented as a strategy to overcome the hydrophobicity limitation of xanthatin, to allow targeted therapy, sustained release, increased stability, increased cell internalization, effectiveness and reduced side effects.

Zhou et al. in 2020 synthesized polydopamine polymeric nanoparticles containing xanthatin where they improved the gastric adhesion and bioavailability of xanthatin. They demonstrated that the nanoparticles could potentially be effective in orally inhibiting gastric cancer.

Zheng X et al. in 2020 obtained polymeric xanthatin micelles functionalized with CD13-specific cyclic peptide (NGR) targeting dendritic cells as a potential drug for the treatment of refractory allergic rhinitis through non-clinical evaluation of intranasal administration in a murine (mouse) model of allergic rhinitis.

Nanocrystal technology is one of the most widely used strategies to improve the solubility and bioavailability of poorly water-soluble drugs. They are nanometer-sized particles of pure drug stabilized by a small amount of suitable surfactants/polymers. To avoid aggregation of particles in the liquid state, freeze-drying is considered one of the techniques per excellence along with spray drying, supercritical fluids, granulation and palletization to improve the long-term stability of colloidal nanoparticles. Drug nanocrystals are a versatile formulation approach to improve the pharmacokinetic and pharmacodynamic properties of poorly water-soluble drugs, offering the opportunity to modify the composition of matter and its physical and/or chemical properties, without altering existing covalent bonds. The decrease in size leads to an increase in surface area, thereby increasing the dissolution rate as well as improving drug solubility, penetrability and adhesion. All these properties contribute to improved drug bioavailability. Compared to other drug delivery systems, nanocrystals are formed directly from drugs and have the advantages of high loading of the active ingredient, easy industrial production and relatively low preparation cost. They can also be administered by a variety of drug delivery routes, such as subcutaneous injection, intravenous injection, oral administration, vaginal administration, transdermal administration and similars. The absence of carrier substances provides a theoretical drug loading of up to 100%, where it is normally between 50-90% (w/w), resulting in satisfactory therapeutic concentrations at lower doses. Toxic side effects resulting from encapsulation/solubilization excipients such as Cremophor RH 40 are also eliminated. Preparation techniques can be divided into: "top-down", "bottom-up" and their combination. Top-down approaches, which mainly involve media milling and highpressure homogenization, employ high mechanical force to convert large drug powders into nano-sized particles. These approaches are simple and fast, do not require organic solvents and are highly reproducible. However, the process is often energy and time consuming, high shear and temperature can cause instability of crystals and subsequent aggregation. There are also concerns about the likelihood of product contamination by grinding media. Bottom-up approaches, which mainly involve solvent-antisolvent precipitation, precisely control the precipitation and crystallization of drugs to achieve the desired nanometric particles, and these approaches are favorable in terms of small and narrow particle size distribution.

*In situ* gelification has been developed over the years for contraception or for the treatment of bacterial, fungal and sexually transmitted infections. It allows controlled and sustained drug release, reduced frequency of administration, lower doses are required, as well as increased bioavailability of the drug, and reduced side effects. In particular, *in situ* thermogelification employs systems that take the form of liquids which may be introduced or deposited on the surface of the body in a minimally invasive technique before solidifying or gelling at body temperature in the target tissue, organ or body cavity. *In situ*-forming polymeric matrices provide advantages over other systems that require surgical implantation procedures and sometimes even need to be removed at the end of treatment. The *in situ* gelling system formulation (thermosensitive hydrogel) is used among other routes for vaginal drug delivery.

Compared to other cancers, therapeutic agents can be administered locally in the vaginal mucosa, and cervical cancer is an excellent option for localized drug delivery, as is the case with systems such as *in situ* gelation. Sustained, localized administration of anti-tumor drugs in the female reproductive tract can avoid the adverse effects associated with systemic administration, improve efficacy by ensuring adequate drug concentration at the site of action, and allow convenient self-administration.

There are currently no commercial formulations containing xanthatin approved by any regulatory authority. Despite the existence of the above formulations, there are no state-of-the-art injectable formulations of xanthatin as an antitumoral that contain nanocrystals of xanthatin without the use of organic solvents that confer high solubility, bioavailability, and effectiveness that is easily scalable. Similarly, there is no formulation of xanthatin in the form of an *in situ* gelable thermosensitive hydrogel containing xanthatin nanocrystals for localized therapy and anti-tumor application. Therefore, the design of new, effective and easy-to-use xanthatin formulations is of important clinical value.

### BRIEF DESCRIPTION OF THE INVENTION

The present disclosure relates to pharmaceutical compositions containing xanthatin as an active pharmaceutical ingredient. In particular, one aspect of the present invention relates to a new process for the preparation of xanthatin nanocrystals. Another aspect of the present invention is a nanocrystal composition prepared according to the described process, comprising xanthatin, a surfactant and a cryoprotectant.

Another aspect described, is a pharmaceutical composition comprising the composition of xanthatin nanocrystals, in conjunction with one or more pharmaceutically acceptable excipients.

A particular aspect of the present invention is a pharmaceutical composition formulated for injectable administration. Additionally, another aspect of the present invention is the use of the injectable formulation in the treatment of colon cancer, lung cancer, breast cancer, cervical cancer, and stomach cancer. In another aspect, a method is described for treating with the injectable formulation a patient suffering from colon, lung, breast, cervical, uterine, or stomach cancer.

Another particular aspect of the present invention is a pharmaceutical composition formulated as an *in situ* gelable thermosensitive hydrogel.

Additionally, another aspect of the present invention is the use of the thermosensitive hydrogel formulation in the treatment of cervical, uterine, colorectal, and ocular cancers. In another aspect, a method for treating a patient suffering from cervical, uterine, colorectal, or ocular cancer with the *in situ* gelable thermosensitive hydrogel is described.

### BRIEF DESCRIPTION OF THE FIGURES

The figures concern freeze-dried xanthatin nanocrystals of example 2 in accordance with the present invention.
Figure 1. Crystal images of: A) isolated xanthatin and B) nanocrystallised xanthatin.

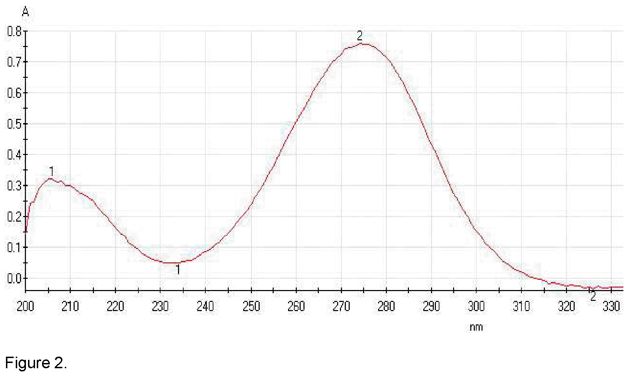

Figure 2. UV spectra, recorded in the 200-400 nm range of nanocrystallised xanthatin.

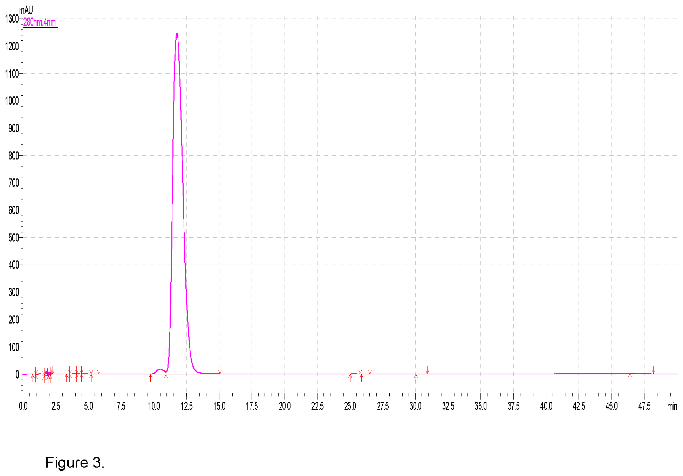

Figure 3. Chromatograms of xanthatin nanocrystals determined by HPLC, recorded at A) 210 nm and B) 280 nm.

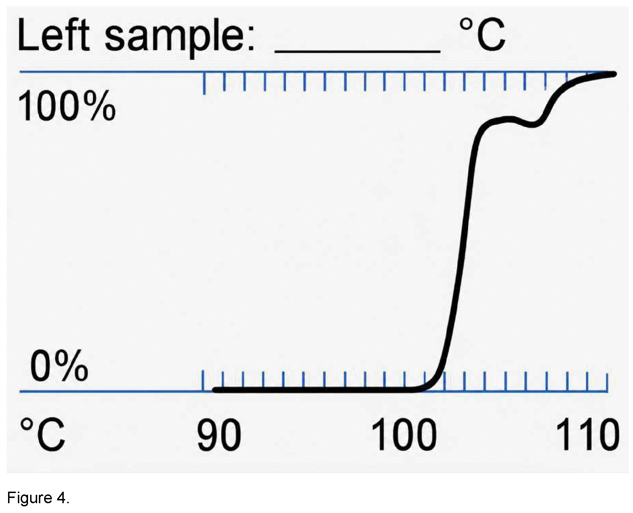

Figure 4. Melting point determination.

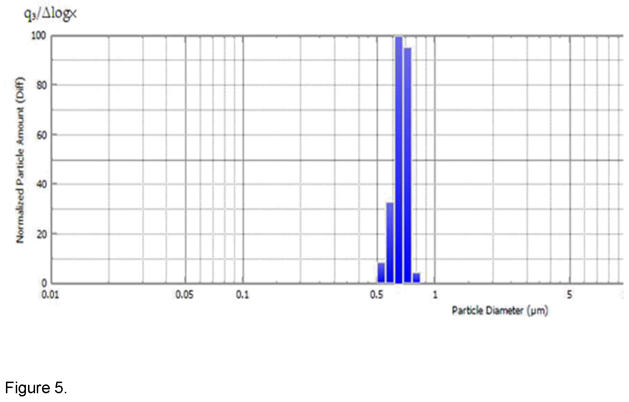

Figure 5. Particle diameter distribution.

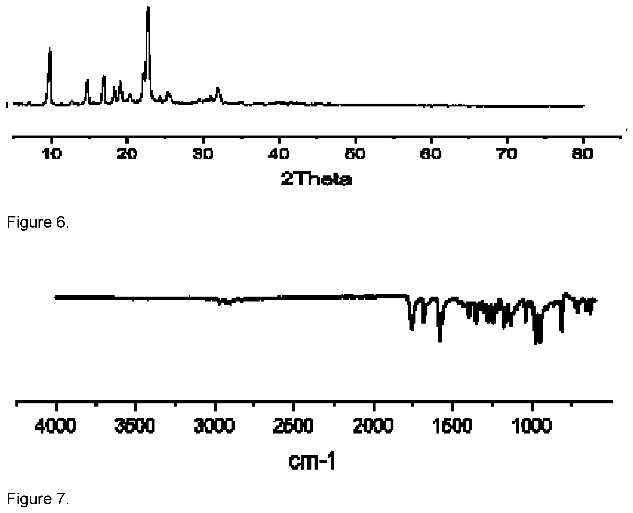

Figure 6. X-ray diffraction spectra.
Figure 7. Fourier transform infrared spectroscopy (FTIS).

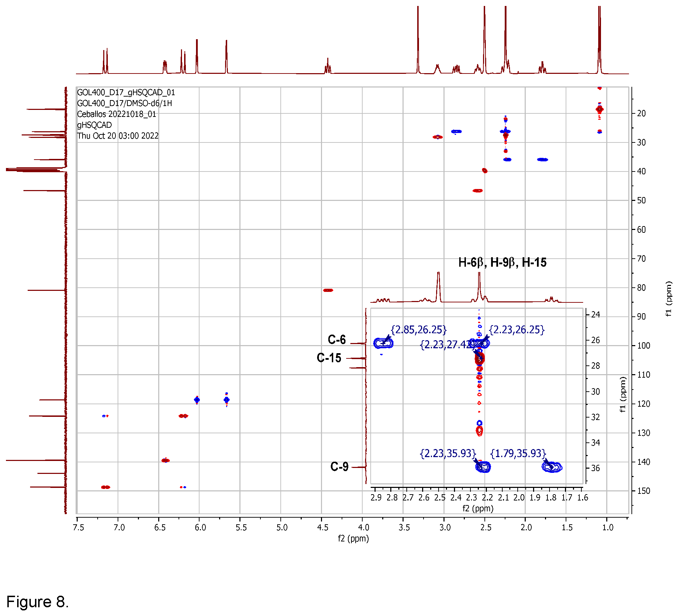

Figure 8. Heteronuclear single quantum correlation (HSQC) spectra of xanthatin nanocrystals. An enlargement of the aliphatic region of the spectrum is shown.

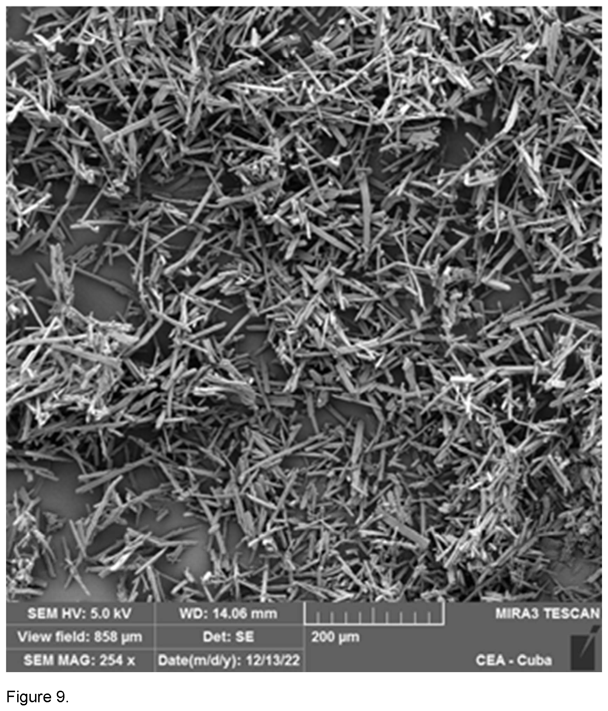

Figure 9. Scanning electron microscopy (SEM).
Figure 10. Effect of commercial xanthatin (XCtrol), isolated xanthatin (XAisl) and nanocrystallized xanthatin (XNano) on cell viability in A) HT-29 and B) Hela cell lines at 24 h. Medium supplemented with 1% fetal bovine serum (FBS) was used as a viability control (Ctrol).
Figure 11. Effect of nanocrystallised xanthatin on cell cycle progression in HT-29 colon cancer cells by flow cytometry. 1% FBS medium was used as an untreated control (Ctrol) and Docetaxel was used as a positive control (DTX). Cells were treated with a concentration of 10 and 20 µM of nanocrystallised xanthatin and isolated xanthatin and 10 µM of commercial xanthatin for 48h. DMSO was used as a control vehicle treatment. Data represent the percent distribution of cells in each phase of the cycle. XCtrol: commercial xanthatin, XAisl: isolated xanthatin, XNano: nanocrystallized xanthatin.
Figure 12. *In vivo* anti-tumour effectiveness of the nanocrystal injectable: A) Tumour volume assessment, and B) tumour weight assessment.
Figure 13. *In vivo* antitumour effectiveness of realisation example number 6 of the *in situ* gelation formulation containing xanthatin nanocrystals of realisation example number 2: A) Tumour weight assessment and B) tumour inhibition rate (%) assessment.

### DETAILED DESCRIPTION OF THE INVENTION

The aim of the present invention is to obtain xanthatin nanocrystals by the antisolvent precipitation method, to dry the suspension of the nanocrystals, with the freeze-dried nanocrystals, and to formulate an injectable and an *in situ* gelation comprising the freeze-dried nanocrystals.

The preparation of the xanthatin nanocrystals of the present invention is carried out by the following technical scheme:
(1) a 0.3% - 10% (w/v, g/ml) portion of xanthatin is dissolved in organic phase as ethanol, solution A;
   The organic phase mentioned above is selected from one or more of dimethyl sulfoxide, propylene carbonate, acetonitrile, acetone, dimethylformamide, tetrahydrofuran, methylpyrrolidone, hexamethylphosphoramide, methanol, ethanol or their combination.
(2) perform sterilization treatment of solution A.
(3) 0.01% -10% (w/v, g/ml) of the stabilizing agent or surfactant is dissolved in water as a dispersed medium, solution B;

The above surfactant is selected from one or more of Tween 20, Tween 40, Tween 50, Tween 60 and Tween 80, poloxamer, phosphatidylcholine or lecithin, gum arabic, gum tragacanth, sodium lauryl sulphate, hydroxypropyl methylcellulose, hydroxyethylcellulose, ethylhydroxyethylcellulose or carboxymethylcellulose; PVP polyvinylpyrrolidone, for example a PVP with a molecular weight in the range of 10,000 to 100,000 Daltons, such as 50,000 to 60,000; salts of bile acids, such as salts of deoxycholic acid, taurocholic acid or glycocholic acid, for example a sodium salt of a bile acid, such as sodium taurocholate, sodium deoxycholate or sodium glycocholate; ethoxylated 12 OH stearic acid (for example Solutol HS15), Pluronic, Tetronic or Lutrol surfactants such as Lutrol F68 or Lutrol F127.

The aqueous phase may contain a single stabilizer or a mixture of two or more stabilizers. (4) 0,01% - 10% (w/w, g/g) of freeze-drying protectant is added to the above solution in relation to the amount of xanthatin used (Solution C).

One of the following is the freeze-drying protectant: glucose, lactose, mannitol, sucrose, trehalose.
(5) carry out the sterilization treatment of solution C.
(6) obtain suspension D by adding A over C under stirring for 5 - 30 min.
(7) remove organic solvent and obtain aqueous suspension of nanocrystals.
(8) freeze suspension D between -40°C and -80°C, and subsequent sublimation by heating between 5°C and 50°C, lyophilized formulations of xanthatin nanocrystals are obtained. In addition to freeze-drying, drying of nanocrystals can be carried out by spray drying, fluidized bed drying, pelletisation, granulation, supercritical fluid drying.

The preparation of injectable formulations follows the following steps.

(9) Lyophilised formulations of xanthatin nanocrystals are added to saline or glucose to re-dissolve for intravenous injection by infusion to be administered at doses of 0.5-2 mg/kg.

The preparation of *in situ* gelled formulations follows the following steps.

(10) slowly add the mucoadhesive polymer at a concentration between 0.1-5% (w/v, g/v) in cold deionized water (between 4-15°C) under slow stirring, solution E.

The mucoadhesive polymer mentioned above is selected from one or more of carbopol 940, HPMC (hydroxypropyl methyl cellulose), NaCMC (sodium carboxymethyl cellulose), Polycarbophil AA1, HPC (hydroxypropyl cellulose), sodium alginate, guar gum.

(11) then add the heat-sensitive polymer at a concentration of 15-25% (w/v, g/v) to solution E under stirring, and allow to dissolve at 4°C for 8 to 16h. solution F.

The above-mentioned thermosensitive polymer is selected from one or more of polaxamer 407 (Pluronic 127), poloxamer 188 (pluronic F68), methylcellulose, PLA-PEG, PLGA-PEG, polycaprolactone-polyethylene glycol block copolymer.

(12) 0.2-10% portion of lyophilized xanthatin nanocrystals (w/v, g/mL) are dispersed in solution F by gentle mixing. Administer intravaginally between 1-3 mL.

### EXAMPLES

The present invention will now be illustrated, but not limited, by the following examples.

### Example 1.

### Preparation of xanthatin nanocrystals

Dissolve 0.5 g of xanthatin in 100 mL of ethanol and sterilize through a 0.2 µm filter, then add this solution under stirring over 400 mL of an aqueous solution previously sterilized by a 0.2 µm filter containing Tween 80 at 1.2 % of the volume of water used and sucrose at 1% of the amount of xanthatin added. Shake for 20 min at 75 rpm. Concentrate under vacuum until the ethanol is removed. The resulting aqueous suspension is frozen at - 80°C for 3h and freeze-dried for 48h at 30°C. The particle diameter can reach 505 nm. A mass of 0,355 g is obtained.

### Example 2.

### Preparation of xanthatin nanocrystals

Dissolve 0,5 g of xanthatin in 100 mL of ethanol and perform sterile filtration through a 0,2 µm filter, then add this solution under stirring over 400 mL of a previously filter-sterilized aqueous solution of 0,2 µm of Tween 80 at 1% of the volume of water used and mannitol at 1% of the mass of xanthatin added. Shake for 20 min at 100 rpm. Concentrate under vacuum until the ethanol is removed. The resulting aqueous suspension is frozen at -80°C for 2h and freeze-dried for 24h at -40°C. The particles can have a diameter of up to 1,5 mm. The particle diameter can reach 669 nm. A mass of 0,386 g is obtained.

### Example 3.

### Preparation of xanthatin nanocrystals

Dissolve 0.5 g of xanthatin in 50 mL of dimethyl sulfoxide (DMSO) and sterilize through a 0.2 µm filter, then add under stirring 400 mL of a previously filter-sterilized aqueous solution of 0.2 µm Tween 80 at 0.8% of the volume of water used and mannitol at 1% of the mass of xanthatin added. Shake for 20 min at 100 rpm. Concentrate under vacuum until the DMSO is removed. The resulting aqueous suspension is frozen at -80°C for 3h and freeze-dried for 24h at -20°C. The particle diameter can reach 650 nm. A mass of 0.372 g is obtained.

### Example 4.

### Preparation of xanthatin nanocrystals

Dissolve 0.5 g of xanthatin in 50 mL of dimethyl sulfoxide (DMSO) and sterile filter through a 0.2 µm filter, then add under stirring 400 mL of an aqueous solution of Tween 20 at 1% of the volume of water used and mannitol at 1% of the mass of xanthatin added. Shake for 15 min at 100 rpm. Concentrate under vacuum until the DMSO is removed. The resulting aqueous suspension is frozen at -80°C for 2h and freeze-dried for 24h at -40°C. The particle diameter can reach 685 nm. A mass of 0,368 g is obtained.

### Example 5.

### Preparation of thermosensitive in situ gelling hydrogel containing xanthatin nanocrystals

Dissolve 1 g of HPMC in 50 mL of cold deionized water at 4°C, then add 20 g of Pluronic 127 under stirring, hold for 10h until completely dissolved. Subsequently add 5 g of xanthatin nanocrystals under stirring until fully dispersed. Add sufficient deionized water to make up to 100 mL.

### Example 6.

### Preparation of in situ gelling thermosensitive hydrogel containing xanthatin nanocrystals

Dissolve 2 g of Carbopol in 50 mL of cold deionized water at 4°C. Subsequently add 15 g Pluronic 127 together with 10 g Pluronic F68 under stirring and keep for 12h until completely dissolved. Add 10 g of xanthatin nanocrystals under stirring until completely dispersed. Add sufficient deionized water to make up to 100 mL.

### Example 7.

### Preparation of in situ gelable thermosensitive hydrogel containing xanthatin nanocrystals

Dissolve 2 g of HPMC in 50 mL of cold deionized water at 4°C. Subsequently add 15 g of Pluronic 127 together with 10 g of pluronic F68 under stirring and keep for 12h until completely dissolved. Add 10 g of xanthatin nanocrystals under stirring until completely dispersed. Add sufficient deionized water to make up to 100 mL.

### Example 8.

### Preparation of thermosensitive in situ gelling hydrogel containing xanthatin nanocrystals

Dissolve 1 g of Carbopol in 50 mL of cold deionized water at 4°C, then add 20 g of Pluronic 127 under stirring, hold for 12h until fully dissolved. Subsequently add 5 g of xanthatin nanocrystals under stirring until fully dispersed. Add sufficient deionized water to make up to 100 mL.

### Example 9.

### Physico-chemical characterization of xanthatin nanocrystals

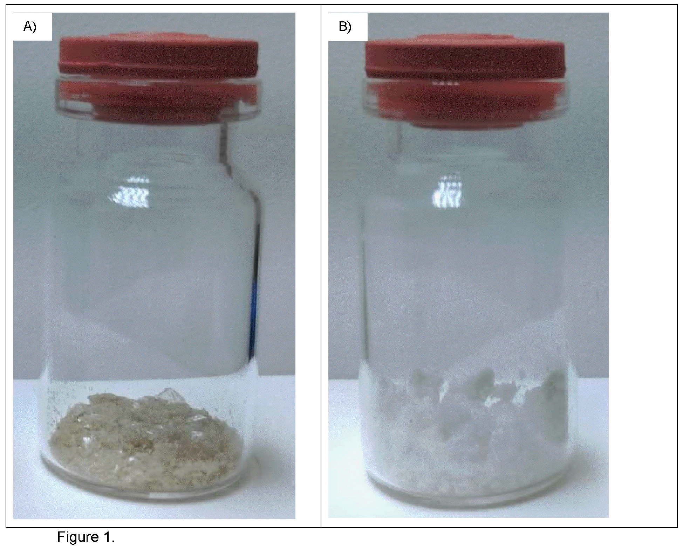

9.1. Determination of organoleptic characteristics in terms of appearance and color. Visual determination of appearance and color was carried out. Figure 1 shows the image of A) isolated xanthatin, and B) freeze-dried nanocrystallised xanthatin.

There are marked differences in the organoleptic characteristics in terms of appearance and color, as the crystals in sample A) have a slight yellow color, in contrast to sample B) where fine white particles are observed.

9.2. Ultraviolet (UV) spectroscopy: Recorded on a Thermo Scientific SPECTRONIC GENESYS spectrophotometer, in the 200-400 nm range, using "pure for chromatographic analysis" quality methanol as blank. The nanocrystallised xanthatin sample was prepared at a concentration of 0.25 mg/mL.

Absorption maxima were observed at 207.2 and 277.1 nm corresponding to the maxima described for this compound.

9.3. High performance liquid chromatography (HPLC) analysis with diode array detector: An HPLC system was used using a Shimadzu equipment with a Supelco reversed phase column (12.5×4.6mm×5µm), with DAD detector. Samples were dissolved in methanol at a concentration of 1 mg/mL and eluted using an acetonitrile/water polarity gradient, as shown in table 1. The injection volume of the samples was 20 µL and the absorbance was read at 210 and 280 nm, methanol was used as blank. A peak purity of 99.99% was determined by chromatographic analysis.

9.4. Melting point determination: The melting point was determined in an automatic melting and boiling point determination apparatus Melting Point M-565. Büchi Labortechnik AG capillaries were used, into which the xanthatin sample was introduced to form a compact column of 4-6 mm height, which was compressed with an M569 sample loader prior to introduction into the apparatus.

The melting point of the nanocrystallized xanthatin is 109.3 ± 0.4°C.

9.5. Particle size determination: The particle size distribution of isolated xanthatin and nanocrystallized xanthatin samples was determined using a SALD 7101 particle analyzer from Shimadzu (Kyoto, Japan) coupled with the WingSALD II 3.0.4 software.

The particle size distribution of the nanocrystallized xanthatin with an average particle size value of 669 ± 43 nm.

9.6. X-ray diffractometry (XRD): X-ray diffraction spectra of isolated xanthatin and nanocrystallized xanthatin were obtained on a MAXIMA-X X-ray diffractometer, XRD-7000 from Shimadzu (Kyoto, Japan) with CuKα radiation at a voltage of 30.0 kV and a current of 30.0 mA. The recordings were performed at 2θ angular interval of (5-80)° and scanning speed 2°/min with continuous scanning. OriginPro 2018 software was used to obtain the spectra.

The characteristic peaks of the crystalline state of xanthatin are observed.

9.7. Fourier transform infrared spectroscopy (FTIR): Isolated xanthatin and the nanocrystals obtained from it were analyzed by means of FTIR in a Prestige 21 IR equipment from Shimadzu (Kyoto, Japan). The test was carried out using the Attenuated Total Reflectance method, using an accessory with a diamond optical window (n=2.4), SPECAC brand, Golden Gate model, with the sample having minimum contact with this window. The scanning range was 4000-600 cm⁻¹, with a mirror speed of 2.0 scans/min and a resolution of 4 cm⁻¹. Prior to sample analysis, a background scan was performed by covering the diamond with the tip for fine powders and then with the accessory (Volatile Cover) for the analysis of liquid samples. The spectrum was obtained from a total of 30 sweeps.

The FTIR spectrum shows the characteristic functional groups for xanthatin where it is evident that there was no degradation during the nanocrystallisation process. The peak near and below 3000 cm⁻¹ corresponds to the C-H bonds, around 1750 cm⁻¹ a typical lactone absorption band is observed. The sharp peaks at about 1670 cm⁻¹ and 1600 cm⁻¹ represent the unsaturated carbonyl system, while the one at 1150 cm⁻¹ represents the methyl group and the peak around 960 cm⁻¹ corresponds to the vinyl.

9.8. Nuclear magnetic resonance (NMR): 1H and 13C NMR spectra were recorded on a Varian VXR-Unity NMR spectrometer at 400 and 100 MHz, as well as multi-pulse experiments (HMBC, HSQC, NOESY and COSY). The solvent used to dissolve the samples was DMSO-d6. Chemical shifts (δ) were expressed in (ppm) and coupling constants (J) in Hz. The δ values are referred to tetramethylsilane as internal reference (TMS).

Correspondence with the signals reported for this compound is shown.

9.9. Scanning electron microscopy (SEM): For the measurement of xanthatin and nanocrystals, a small portion of each sample was deposited in the sample holders with carbon tape, then coated with a 15 nm thick gold layer with the aim of making the study samples conductive. Once this process was concluded the analysis was carried out in the TESCAN Scanning Electron Microscope, model FE-SEM MIRA3 coming from Micra Nanotechnology (Mexico Federal District of Mexico, Mexico) with an Energy Dispersive X-Ray detector (EDS).

The nanocrystals show particles with small needle-like morphology, without agglomerations and with uniform size.

### Example 10.

Cell growth inhibition of nanocrystallized xanthatin assessed using the MTT assay.

The viability of the cultured cells was assessed using the MTT reduction assay. The assay was performed using the method described by Mosmann (1983). HT-29 and HeLa cells were seeded in 96-well plates at 10⁴ cells/ml and 5x10³ cells/ml respectively. The cells were cultured in Dulbecco's modified Eagle's medium (DMEM) supplemented with 10% FBS, 1% glutamine, triple antibiotic (3A), and 25 mM glucose, and maintained at 37°C in a 5% CO₂ incubator until 80-90% confluence. After 24h of incubation, to ensure cell adhesion, the medium was removed and different concentrations (10 and 20 µM) of either commercial xanthatin, isolated xanthatin, and xanthatin nanocrystals, or as a negative control medium supplemented with 1% DMSO, were added. Prior to treatment, the commercial and isolated xanthatin samples were dissolved in DMSO, unlike the nanocrystallized xanthatin that was directly added to the cell culture medium. The prepared plates were incubated for 24 h, and 50 µL MTT (5 mg/ml) per well was added to a final concentration of 1 mg/ml. The cells were incubated for 4 h at 37°C, and the medium was discarded. DMSO (100 µl per well) was added to each well and resuspended to dissolve formazan crystals. Relative cell viability was determined by measuring the absorbance at 540 nm on an ELISA plate reader (Tecan Sunrise MR20-301, TECAN Austria). Absorbance is directly proportional to the level of cell proliferation or viability. $Viability \left(%\right) = \frac{OD values of samples treated with drugs}{OD values of untreated samples} \times 100$

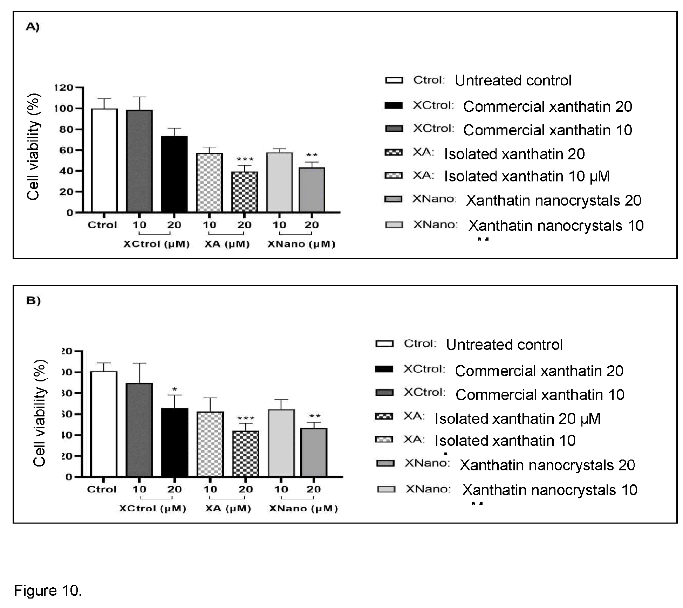

Figure 10 shows the graphs of the results of the effect of xanthatin nanocrystals, commercial xanthatin and xanthatin isolated in the laboratory on the cell viability of human HT-29 colorectal adenocarcinoma (Fig A), and human HeLa cervical carcinoma tumor lines (Fig B). MTT assay was performed as described by Mosmann in 1983 using DMSO as a negative control. It can be seen that the viability inhibition exerted by laboratory isolated xanthatin and xanthatin nanocrystals on both cell lines was significantly higher than that of commercial xanthatin. There were no statistically significant differences in the effect of xanthatin isolated in the laboratory (dissolved in DMSO prior to treatment), with respect to the nanocrystallized xanthatin obtained from it, but added directly to the cell culture medium. This is indicative of the effectiveness of xanthatin nanocrystals by increasing xanthatin solubility through nanocrystallization, since the use of organic solvents such as DMSO, necessary to dilute drugs that are poorly soluble in water, as is the case with xanthatin, prior to treatment in cell cultures, was not required.

### Example 11.

Assay to evaluate the *in vitro* antiproliferative activity of nanocrystallized xanthatin by flow cytometry.

The antiproliferative activity through cell cycling arrest was evaluated in HT-29 cells. Cells were cultured in 6-well plates (2×10⁵ and 1×10⁵ cells/ml, respectively) until 90% confluence was reached. Three xanthatin samples were evaluated: commercial, laboratory-isolated, and nanocrystallized xanthatin at concentrations of 10 and 20 µM. Prior to treatment, both commercial and isolated xanthatin were dissolved in DMSO, unlike nanocrystallized xanthatin, which was added directly to the cell culture medium. Medium supplemented with 1% FBS was used as the control. Docetaxel 2 µM was used as a positive control for cell cycle arrest in G2/M. The treatments were added to the medium supplemented with 1% FBS on the day after seeding the cells on the plates. The cells were incubated for 48 h upon the treatments, harvested, fixed in 100% ethanol, and stored at -20°C. Cell cycle distribution was next assessed using flow cytometry. On the day of the assay, samples were incubated with a PI/Rnase mixture at room temperature and in the dark for 20 min prior to analysis.

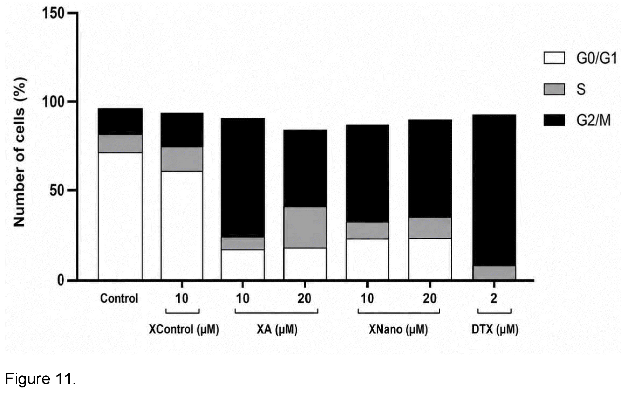

Figure 11 shows the results associated with the effect of commercial xanthatin, isolated xanthatin, and xanthatin nanocrystals on cell cycle progression in the HT-29 human colorectal adenocarcinoma tumor line by flow cytometry analysis. There were no statistically significant differences between the effects of commercial xanthatin and those of the untreated control group in the G2/M phase. There were no statistically significant differences between xanthatin isolated in the laboratory (dissolved in DMSO prior to treatment) and the nanocrystallized xanthatin obtained from it, but added directly to the cell culture medium. The graph shows that both isolated xanthatin (dissolved in DMSO prior to treatment) and nanocrystallized xanthatin (added directly to the cell culture medium) induced cell cycle arrest in the G2/M phase without statistically significant differences compared with the positive control group (docetaxel). This result is indicative of the effectiveness of xanthatin nanocrystals in increasing the solubility of xanthatin, since an organic solvent such as DMSO, which is necessary for substances that are poorly soluble in water (as is the case with xanthatin), was not used prior to treatment.

### Example 12.

### Evaluation of the in vivo antitumor effect of injectable xanthatin nanocrystals

The *in vivo* antitumor effect of the injectable xanthatin nanocrystal was evaluated. For this purpose, CT26WT colon carcinoma cells were inoculated into male BALB/c mice with average weights of 22-24 g. Tumor implantation was performed by inoculating subcutaneously (s.c) in the right flank of each animal 0.2 ml of cell suspension with a total of 5x10⁶ cells/mouse. Four groups of 7 animals each were randomly distributed. The injectable xanthatin nanocrystal was prepared by diluting the nanocrystals in water for injection at a concentration of 4 mg/ml. The treatment schedule was carried out for 10 days at the rate of daily intravenous administration of nanocrystallized xanthatin injectable at 20 mg/kg, placebo of the injectable (without xanthatin nanocrystals), and every 2 days a daily administration of oxaliplatin at 6 mg/kg (four administrations) intraperitoneally. Tumor weight and volume were evaluated. Tumor volume was calculated using the following formula: $Tumor volume = 0 , 5 \times lenght \times {widht}^{2}$

Tumor length and width were measured every three days using calipers.

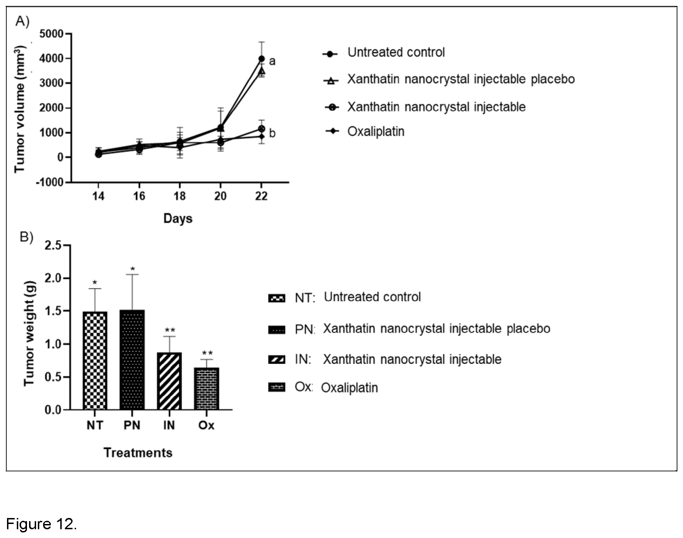

Figure 12 shows the *in vivo* antitumor effect of the injectable nanocrystal in male BALB/c mice inoculated with CT26WT colon carcinoma cells. The graphs show that the intravenously administered nanocrystallized xanthatin injectable inhibits tumor growth by decreasing A) tumor volume, and B) tumor weight in BALB/c mice, with no statistically significant differences compared to oxaliplatin injectable.

### Example 13.

Evaluation of the *in vivo* antitumor effects of *in situ* gelation containing xanthatin nanocrystals.

Evaluation of the *in vivo* antitumor effect of *in situ* gelation containing xanthatin nanocrystals was performed using murine U14 cervical carcinoma cells inoculated into the cervix of female BALB/c mice with an average weights of 22-24 g. Tumor implantation was performed by inoculating the submucosa near the cervix of each animal with 25 µL of cell suspension in PBS, with a total of 4×10⁵ cells/mouse. Four randomly distributed groups of 6 animals per group were formed. The treatment schedule was carried out for 5 days with a daily intravaginal infusion of the thermosensitive hydrogel containing the xanthatin nanocrystals (8.5 µL, 0.850 mg of nanocrystallized xanthatin per mouse) and placebo, and for 3 days with a daily intravaginal infusion of the xanthatin nanocrystals (8.5 µL, 0.850 mg of nanocrystallized xanthatin per mouse) and placebo. The positive control of carboplatin in the intravenous injection (0.675 mg of carboplatin per mouse) was administered for 3 days (once every other day). Tumor weight and tumor inhibition rates were evaluated. Tumor inhibition rate was calculated using the following formula: $TIR = \frac{{A}_{w} - {A}_{x}}{{A}_{c}} \times 100$

Where:
TIR means tumor inhibition rate in %.
AW means the average weight of tumors in the control group.
Ax means the average weight of the tumors of the treated group.

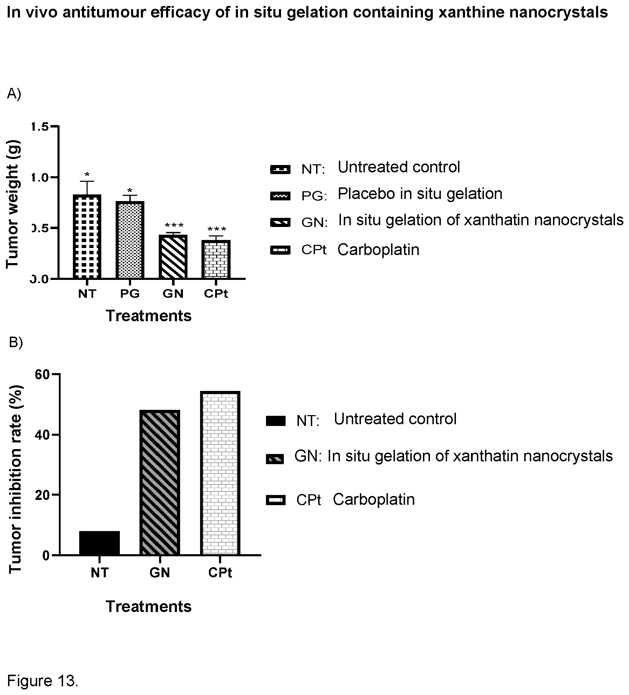

Figure 13 shows the *in vivo* antitumor effect of realization example number 6 of the *in situ* gelation formulation containing xanthatin nanocrystals of realization example number 2, on murine U14 cervical carcinoma cells inoculated into the cervix of female BALB/c mice. A significant decrease in tumor weight was observed in the groups treated with the nanoxanthatin and carboplatin injectable with respect to the placebo and the control groups, with no statistically significant differences between the *in situ* gelation of the xanthatin nanocrystals and the carboplatin injectable. The tumor inhibition rates of the xanthatin nanocrystal hydrogel and injectable carboplatin were 48.2% and 54.4%, respectively.

### References

### Patent documents

JPH09188671A
CA2688486A1
CN110123754A

### Other publications

1. Favier, L.S., et al., Anti-ulcerogenic activity of xanthanolide sesquiterpenes from Xanthium cavanillesii in rats. Journal of Ethnopharmacology 2005. 100: p. 260-267.
2. Ferrer, J.P. and Á.S. Lamar, Blancos mitóticos de drogas naturales y nuevas estrategias para la terapia anti-cáncer. Revista Cubana de Ciencias Biológicas, 2016. 4(3): p. 3-15
3. Geng, Y.-d., et al., Xanthatin mediates G2/M cell cycle arrest, autophagy and apoptosis via ROS/XIAP signaling in human colon cancer cells. Natural product research, 2020. 34(18): p. 2616-2620
4. Jean Legault, Andre Pichette, Serge Lavoie, titular. Sesquiterpene formulations, kits and methods of use thereof. Patente canadiense CA2688486A1. 2 de junio del 2008. [consultado el 6 de abril de 2023] Disponible en: https://patents.qoogle.com/patent/CA2688486A1/en
5. Kovács A, Vasas A, Forgo P, Réthy B, Zupkó I, Hohmann J. Xanthanolides with antitumour activity from Xanthium italicum. Z Naturforsch C J Biosci. 2009 May-Jun;64(5-6):343-9
6. Li, W. D., Wu, Y., Zhang, L., Yan, L. G., Yin, F. Z. et al. Characterization of xanthatin: anticancer properties and mechanisms of inhibited murine melanoma in vitro and in vivo. Phytomedicine. 2012., 20(10): 865-873.
7. Liu, M., Xiao, C. Q., Sun, M. W., Tan, M. J., Hu, L. H., & Yu, Q. Xanthatin inhibits STAT3 and NF-κB signalling by covalently binding to JAK and IKK kinases. Journal of Cellular and Molecular Medicine. 2019; 23(6), 4301-4312.
8. Lu, L., Xu, Q., Wang, J., Wu, S., Luo, Z., & Lu, W. Drug nanocrystals for active tumor-targeted drug delivery. Pharmaceutics. 2022; 14(4), 797.
9. Lu, Y., Li, Y., & Wu, W. Injected nanocrystals for targeted drug delivery. Acta Pharmaceutica Sinica B. 2016; 6(2), 106-113.
10. Nibret, E., Youns, M., Krauth-Siegel, R. L. and Wink, M. Biological activities of xanthatin from Xanthium strumarium leaves. Phytother Res. 2011 25(12): 1883-1890.
11. Ramirez-Erosa, I., Huang, Y., Hickie, R. A., Sutherland, R. G. and Barl, B. Xanthatin and xanthinosin from the burs of Xanthium strumarium L. as potential anticancer agents. Can J Physiol Pharmacol. 2007; 85(11): 1160-1172.
12. Ran, Q., Wang, M., Kuang, W., Ouyang, J., Han, D., Gao, Z., & Gong, J. Advances of Combinative Nanocrystal Preparation Technology for Improving the Insoluble Drug Solubility and Bioavailability. Crystals. 2022; 12(9), 1200.
13. Shen, M., Zhou, X. Z., Ye, L., Yuan, Q., Shi, C., Zhu, P. W., ... & Shao, Y. (2018). Xanthatin inhibits corneal neovascularization by inhibiting the VEGFR2 mediated STAT3/PI3K/Akt signaling pathway. International journal of molecular medicine. 2018; 42(2), 769-778.
14. Takeda, S., et al., (-)-Xanthatin induces the prolonged expression of c-Fos through an N-acetyl-L-cysteine (NAC)-sensitive mechanism in human breast cancer MDA-MB-231 cells. Journal of Toxicological Sciences 2013. 38(4): p. 547-557
15. Tao, L., et al., Xanthatin triggers Chk1-mediated DNA damage response and destabilizes Cdc25C via lysosomal degradation in lung cancer cells. Toxicology and Applied Pharmacology, 2017. 337: p. 85-94.
16. Tao, L., Fan, F., Liu, Y., Li, W., Zhang, L. et al. (2013). Concerted suppression of STAT3 and GSK3beta is involved in growth inhibition of non-small cell lung cancer by Xanthatin. PLoS One. 2013; 8(11): e81945.
17. Vasas, A. and J. Hohmann, Xanthane sesquiterpenoids: structure, synthesis and biological activity. Natural Products Reports 2011. 28: p. 824-842.
18. Xia Ming, Wang Cheng, Zhao Miaoqing, Liu Chengcheng, Sol Chao, Zheng Xue, , titular. A kind of targeting is in the Xanthatin nano-micelle and preparation method and application of dendritic cells. Patente china CN110123754A. 24 de junio de 2019. [consultado el 6 de abril de 2023] Disponible en: https://patents.google.com/patent/CN110123754A/en?q=(xanthatin)&oq=xantha tin
19. Yan C, Li H, Wu Y, Xie D, Weng Z, Cai B, Liu X, Li W, Chen Z. Determination of xanthatin by ultra high performance liquid chromatography coupled with triple quadrupole mass spectrometry: application to pharmacokinetic study of xanthatin in rat plasma. J Chromatogr B Analyt Technol Biomed Life Sci. 2014 Feb 1;947-948:57-61.
20. Yoichi Sato, Tetsuya Otsubo, Tomihiko Higuchi, titular. Xanthatin-containing antitumor medicinal composition. Patente japonesa JPH09188671A. 9 de enero de 1996. [consultado el 6 de abril de 2023]. Disponible en: https://patents.google.com/patent/JPH09188671A/en
21. Yu, Y., Yu, J., Pei, C. G., Li, Y. Y., Tu, P. et al. Xanthatin, a novel potent inhibitor of VEGFR2 signaling, inhibits angiogenesis and tumor growth in breast cancer cells. Int J Clin Exp Pathol. 2015; 8(9): 10355-10364.
22. Zhang, L., Ruan, J., Yan, L., Li, W., Wu, Y. et al. Xanthatin induces cell cycle arrest at G2/M checkpoint and apoptosis via disrupting NF-kappaB pathway in A549 non-small-cell lung cancer cells. Molecules. 2012a. 17(4): 3736-3750.
23. Zhang, L., et al., Xanthatin Induces G2/M Cell Cycle Arrest and Apoptosis in Human Gastric Carcinoma MKN-45 Cells. Planta Medica, 2012b. 78: p. 890-895
24. Zheng X, Sun C, Yu R, Chu X, Xu J, Liu C, Zhao M, Xu X, Xia M, Wang C. CD13-specific ligand facilitates Xanthatin nanomedicine targeting dendritic cells for therapy of refractory allergic rhinitis. Int J Pharm. 2020 Mar 15;577:119034. doi: 10.1016/j.ijpharm.2020.119034. Epub 2020 Jan 25. PMID: 31991183.
25. Zhou Y, Zhu X, Lin S, Zhu C, Wu L, Chen R, Chen Z, Li W. A Novel Nanoparticle Preparation to Enhance the Gastric Adhesion and Bioavailability of Xanthatin. Int J Nanomedicine. 2020 Jul 14;15:5073-5082.

## Claims

1. A method of preparing nanocrystals with improved solubility, **characterized in that** the preparation procedure in powder form comprises the following steps:
a) dissolution of xanthatin in an organic solvent.
b) dissolution of surfactant in water together with the cryoprotectant.
c) transfer of the mixture obtained in a) to the aqueous solution obtained in b), stirring and continuous mixing for 5 to 30 minutes.
d) elimination of the organic solvent to obtain an aqueous suspension of nanocrystals.
e) drying of the product until a powder is obtained.

2. The method of preparing nanocrystals according to claim 1, **characterized in that** the organic solvent used in step a) is dimethyl sulfoxide, propylene carbonate, acetonitrile, acetone,dimethylformamide,tetrahydrofuran,methylpyrrolidone,hexamethylphosphoram ide, methanol, ethanol or a combination thereof.

3. The method of preparing nanocrystals according to claim 1, **characterized in that** ratios of 0.3% - 10% (w/v) of xanthatin are used.

4. The method of preparing nanocrystals according to claim 1, **characterized in that** the surfactant used in step b) is added in concentrations between 0.01-10% and is selected from the group comprising Tween 80, Tween 20, Tween 40, Tween 60 and Tween 50, poloxamer, hydroxypropylmethylcellulose, hydroxyethylcellulose,30 ethylhydroxyethylcellulose or carboxymethylcellulose; PVP polyvinylpyrrolidone; salts of bile acids; 12 OH ethoxylated stearic acid, Pluronic, Tetronic or a combination thereof.

5. The method of preparing nanocrystals according to claim 1, **characterized in that** the cryoprotectant used in step b) is added from 0.5-10% (w/w, g/g) and is selected from the group consisting of lactose, mannitol, sucrose, trehalose, fructose, glucose, sodium alginate, gelatin or a combination thereof.

6. The method of preparing nanocrystals according to claim 1, **characterized in that** the drying step in step e) is carried out by freeze-drying, spray drying, fluidized bed, pelletization, granulation, supercritical fluids.

7. A freeze-dried nanocrystal composition with enhanced solubility prepared by the method according to claim 1, **characterized in that** it comprises xanthatin, surfactant and a cryoprotectant.

8. The freeze-dried nanocrystal composition according to claim 7, **characterized in that** the average size of the nanocrystals is between 100 - 700 nm.

9. The freeze-dried nanocrystal composition according to claims 7, **characterized in that** xanthatin is present between 85-99.5%.

10. The freeze-dried nanocrystal composition according to claim 7, **characterized in that** the surfactant is present in concentrations between 0.01 -10 % and is selected from the group comprising Tween 80, Tween 20, Tween 40, Tween 60 and Tween 50, poloxamer, phosphatidylcholine or lecithin, gum arabic, gum tragacanth, sodium lauryl sulfate hydroxypropyl methylcellulose, hydroxyethylcellulose, 30 ethylhydroxyethylcellulose or carboxymethylcellulose; PVP polyvinylpyrrolidone; salts of bile acids; 12 OH ethoxylated stearic acid, Pluronic, Tetronic or a combination thereof.

11. The freeze-dried nanocrystal composition according to claim 7, **characterized in that** the cryoprotectant is present between 0.4 - 5 %, is selected from the group comprising lactose, mannitol, sucrose, trehalose, fructose, glucose, sodium alginate, gelatin or a combination thereof.

12. A pharmaceutical composition comprising the lyophilized nanocrystal composition according to claims 7 to 11 and pharmaceutically acceptable excipients.

13. The pharmaceutical composition according to claim 12, wherein the composition is formulated for intravenous administration and is formulated in powder form for reconstitution with saline or glucose for infusion.

14. The pharmaceutical composition according to claim 13, wherein the composition is formulated for direct tumor injection or infusion or infusion.

15. The lyophilized nanocrystal composition according to claim 11, wherein the composition is formulated for vaginal, rectal or ocular administration and comprises a thermosensitive hydrogel.

16. The thermosensitive hydrogel according to claim 15 comprising:
- a 0.2-10% portion of lyophilized nanocrystals.
- a thermosensitive polymer
- a mucoadhesive excipient

17. The thermosensitive hydrogel according to claim 15, **characterized in that** the thermosensitive agent is used in concentrations between 15 and 25% (w/v, g/v) and is selected from the group consisting of poloxamers; PLA-PEG copolymers; PLGA-PEG copolymers; methylcellulose; polycaprolactone block copolymer; preferably poloxamers, and more preferably is poloxamer 407 and/or poloxamer 188 or a combination thereof.

18. The thermosensitive hydrogel according to claim 15, **characterized in that** the mucoadhesive excipient is used in a concentration between 0.1 and 5 % (w/v, g/v) and is selected from the group consisting of carbopol 940, HPMC (hydroxypropylcellulose), NaCMC (sodium carboxymethylcellulose), Polycarbophil AA1, HPC (hydroxypropylcellulose), sodium alginate, guar gum.

19. A pharmaceutical composition as defined in any one of claims 12 to 18, for use in the treatment of cancer.

20. Use of a pharmaceutical composition according to claims 13 and 14 for the treatment of colon, lung, breast, cervical uterine, stomach cancer.

21. A method of treating a disease in a patient which method comprises administering to said patient a therapeutically effective amount of the pharmaceutical composition according to claims 13 and 14, wherein the disease is selected from: colon, lung, breast, cervical uterine, stomach cancer.

22. Use of a pharmaceutical composition according to claims 15 and 18 for the treatment of cervical uterine, colorectal, ocular cancer.

23. A method of treating a disease in a patient which method comprises administering to said patient a therapeutically effective amount of the pharmaceutical composition according to claims 15 and 18, wherein the disease is selected from: cervical uterine, colorectal, ocular cancer.
